Europäisches Patentamt

European Patent Office

Office européen des brevets

(19)

(11) Publication number: **0 084 190**
**A2**

# (12) EUROPEAN PATENT APPLICATION

(21) Application number: **82201446.0**

(22) Date of filing: **16.11.82**

(51) Int. Cl.³: **A 61 K 31/35**
**A 61 K 9/14, A 61 L 15/03**

(30) Priority: **08.12.81 GB 8136909**

(43) Date of publication of application:
**27.07.83 Bulletin 83/30**

(84) Designated Contracting States:
**DE GB NL SE**

(71) Applicant: FISONS plc
Fison House Princes Street
Ipswich Suffolk IP1 1QH(GB)

(72) Inventor: Murphy, Finnbarr
23 Main Street
Woodhouse Eaves Leicestershire(GB)

(74) Representative: Craig, Christopher Bradberry et al,
Fisons plc 12 Derby Road
Loughborough Leicestershire LE11 0BB(GB)

(54) Medicinal composition, package and method of making the composition.

(57) There is described a mixture of cromoglycic acid, or a pharmaceutically acceptable salt thereof, and substantially dry particles of a water-insoluble hydrophilic macromolecular material capable of undergoing limited swelling in fluid to form discrete gel particles, the swellability of the macromolecular material being such that the particles can absorb low molecular weight constituents of blood plasma but cannot absorb to any material extent fibrinogen or other substances of the same or higher molecular weight.

There are also described a package containing the mixture, methods of making the mixture and use of the mixture to treat wounds.

EP 0 084 190 A2

## COMPOSITION, PACKAGE AND METHOD OF MAKING THE COMPOSITION

This invention relates to novel mixtures, methods for preparing them and pharmaceutical compositions containing them.

Sodium cromoglycate is known, e.g. from British Patent Specification No 1,444,905, to be useful in the treatment of allergic conditions, and notably asthma. We have now found a means of formulating cromoglycic acid and salts thereof, for application to wounds and other skin lesions, in admixture with macromolecules some of which have been known as gel filtration chromatographic materials.

According to the invention we provide a mixture of cromoglycic acid, or a pharmaceutically acceptable salt thereof, and substantially dry particles of a water-insoluble hydrophilic macromolecular material capable of undergoing limited swelling in fluid to form discrete gel particles, the swellability of the macromolecular material being such that the particles can absorb low molecular weight constitutents of blood plasma but cannot absorb to any material extent fibrinogen or other substances of the same or higher molecular weight.

The mixture may be in further admixture or conjunction with a dermatologically suitable carrier. The preparation of the invention may be used for treating any rupture of the skin surface, e.g. ulcers, small wounds or burns, whether external or internal, from which there is a discharge. The preparation of the invention may be used for treating humans or animals; the animals may be commercially reared animals.

In addition to low molecular weight salts, for example NaCl, KCl and $CaCl_2$, and amino acids, urea and glucose, the low molecular weight constituents of blood plasma include the so-called thrombocyte factors which influence the first phase of the blood coagulation process. Examples of such low molecular weight active substances include prostaglandins, serotonin, adrenaline, adenosine diphosphate (ADP), certain exo- and endo-toxins having hydrolytic properties and divalent cations such as calcium or magnesium.

The macromolecular material may be such that high molecular weight degradation products of the fibrinogen, for example those having a molecular weight of over 270,000, preferably over 165,000, and possibly also those having a molecular weight of over 85,000, or even those having a molecular weight of over 50,000, are completely or partly excluded from the particles. The macromolecular material may also be such that, in addition to the low molecular weight constituents of blood plasma also substances having larger molecular weights (although smaller than fibrinogen) are permitted to penetrate completely or partially into the water-swollen particles. Examples of such relatively low molecular weight substances are relatively low molecular weight proteins and polypeptides, for example those having a low molecular weight in the region of up to approximately 5,000 or up to approximately 20,000 or, in certain instances, up to approximately 40,000.

The macromolecular material is selected so that particles thereof in a water-swollen state could be used in a column for conventional gel filtration purposes, thereby making possible, by means of gel filtration techniques, the complete or partial separation of molecules of, for example, the size of fibrinogen from small or smaller molecules of the afore-mentioned type. We particularly prefer the water-swollen macromolecular material to comprise a three-dimensional network.

The particles according to the invention are generally soft and will not adhere to the surface of the rupture, even when saturated with fluid from the discharging surface, thereby enabling the particles to be removed painlessly. The gel formed can be readily removed by means of a spatula or may be washed away with a physiological saline solution.

The water-insoluble hydrophilic macromolecular material capable of undergoing limited swelling to form a gel may contain hydroxyl groups. The hydrophilic properties of the macromolecular material may also be obtained by means of carboxyl groups, which are perferably present in the form of a physiologically tolerable salt. In addition, or alternatively, the macromolecular material may contain amino groups and/or sulphonic acid groups, each of which is preferably in the form of physiologically tolerable salts.

The hydrophilic property of the macromolecular material is advantageously such that 1g of the dry material, when swelling in the presence of water, absorbs at least 0.5g, preferably at least 1g, of water. In general, 1g of the dry macromolecular material should absorb less than 30g, preferably less than 25g (advantageously less than 20g and especially, less than 15g) of water. If the gel-forming particles used in the mixture comprise, for example, dextran or carboxymethyl-dextran or starch or hydroxyethyl starch crosslinked by means of epichlorohydrin in alkaline aqueous solution to form a water-insoluble but swellable three-dimensional network, a particularly good result is obtained if the degree of crosslinking corresponds to a water absorption ability of approximately 1.5 to 10g, e.g. 2 to 5g, of water per gram of dry macromolecular material.

The macromolecular material preferably has a high swelling rate when it comes into contact with water or with the aqueous fluid on the skin surface to be treated. The particles should normally absorb water and swell at a rate which is sufficiently high that fibrin and fibrin coagulum cannot be formed by the influence of the enzyme thrombin etc in the zone adjacent to the discharging surface. Since fibrin is formed at different rates with different people there is conveniently selected a swelling rate for the particles which is sufficiently high for use with people having a high rate of fibrin formation. A satisfactory swelling rate in physiological saline solution or in water is such that 1 gram of the dry particles of macromolecular material absorbs in 5 seconds more than 0.02, preferably more than 0.1, advantageously more than 0.2., and especially more than 0.5g of water. The preferred upper limit on the amount of water absorbed under the above conditions is 5g, more especially 3g, and, in particular 2g.

The water-insoluble hydrophilic material capable of under-going limited swelling to form a gel preferably comprises a three-dimensional network held together by bonds of a covalent nature. Thus, for example, the macromolecular material may comprise hydroxyl group-containing macromolecules cross-linked by means of bridges connected to said polymer molecules by ether bonds. The bridges may be straight or branched aliphatic saturated hydrocarbon chains substituted by one or more hydroxyl groups and containing 3 to 20 carbon atoms, preferably 3 to 10 carbon atoms, said chains being optionally interrupted by one or more oxygen atoms. The product is insoluble, but swellable in water to form a gel, said gel in a fully water-swollen state advantageously containing more than 30, preferably more than 50, especially more than 60% by weight of water and less than 97, preferably less than 95, especially less than 90% by weight of water. When the cross-linked product contains hydroxyl-groups and/or carboxyl groups and/or sulphonic acid groups and/or amino groups, said groups may make up more than 5, preferably more than 10, advantageously more than 20% by weight and less than 60, preferably less than 50, advantageously less than 40% by weight of the dry particles. The cross-linked macromolecules containing hydroxyl groups may be polymeric or polymerised carbohydrates or sugar alcohols or other macromolecular materials containing hydroxyl groups. Examples of suitable macromolecular materials which are insoluble, but swellable in water, are materials obtained, for example, by cross-linking any one or more of dextran, hydroxyethyl dextran, carboxymethyl dextran, sulphopropyl dextran, diethylaminoethyl dextran, hydroxyethyl cellulose, carboxymethyl cellulose, starch, hydroxyethyl starch, carboxymethyl starch, any other polysaccharides and polysaccharide derivatives, polyvinyl alcohol, saccharose, sorbitol or mannitol in alkaline aqueous solution by means of bridge forming substances using conventional means known per se. A preferred macromolecule is dextran cross-linked with epichlorhydrin, e.g. the material known as dextranomer.

A macromolecular product capable of being slowly broken down enzymatically to water-soluble fragments may also be used. Thus, for example, it is possible to use starch, hydroxyethyl starch, carboxymethyl starch, or another starch derivative which has been crosslinked in the aforementioned manner by a bridge-forming agent which is at least bifunctional, the degree of total substitution for the starch being selected so that alpha-amylase is able to break glucosidic linkages in the three-dimensional network to such an extent that, if the gel particles are not removed after the cleansing process, they gradually disappear as water-soluble fragments as a result of the influence of alpha-amylase in the aqueous body fluid.

The dry particles advantageously have an average particle size within the range of 10-10000 microns, preferably 20-500 microns, especially 30-400 microns, and more preferably 50-300 microns.

The macromolecular materials may be used in the form of spherical particles (e.g. obtained by so-called bead polymerisation processes) or particles having irregular configuration (e.g. obtained by so-called bulk polymerisation and grinding). Examples of some macromolecular materials which can be used according to the invention provided that they have the required swelling properties are found in British Patent specification Nos 854,715; 936,039; 974,054; and 1,013,585.

The preparation of the invention may contain the macromolecular material in the form of a coherent layer, and may be applied to the required surface in such a manner as to enable the aqueous fluid on said surface to be absorbed by the material, whereafter the layer of material with fluid absorbed therein is removed from said surface. The process can be repeated one or more times.

The carrier used in the preparation of the invention should be compatible with the cromoglycic acid, or the pharmaceutically acceptable salt thereof, and with the macromolecular material and may be selected from inert fillers and other dermatologically acceptable additives, for example soap, invert soap and other conventional skin cleansing agents. Perfumes, wetting agents and disinfectants may also be used.

We prefer to use a pharmaceutically acceptable salt of cromoglycic acid, e.g. the disodium salt thereof, which is commonly known as sodium cromoglycate.

The invention also includes a pack which contains the mixture of the invention or its components and one or more protective layers for retaining the mixture when it is applied to a wound. Thus, such a pack may advantageously be used when it is desired to apply a layer of the mixture to a wound surface and to fix it more effectively to the application site by covering the layer with such an associated protective layer, for example, a film of an elastic plastic material, which film is preferably provided with small openings to admit breathing to a certain degree.

Similarly, for treating leg sores a pack in accordance with the invention contains a protective layer in the form of a plastic tube which can be inserted over the leg portion in question. The tube is then tightened below as well as above the leg sore, the space between the tube and the leg being filled with the mixture.

The pack in accordance with the invention may also comprise a package which contains the mixture and means for spraying or painting a film-forming liquid dressing over the mixture. The pack may be a bag containing the mixture which can be used as a bandage.

The mixture may be mixed with or incorporated in different inert materials to form, for example, dressings or bandages. Thus, for example, particles may be mixed in dry or wet conditions with different fibrous materials, for example, cellulose fibres or paper pulp. The weight proportion between particles and fibrous material can be varied within broad limits, for example between 5:1 and 1:5.

The mixture and any additive, for example fibres, can also be mixed with dermatologically acceptable binders, for example polyethylene glycols, carboxymethyl cellulose or gum arabic. These binders are preferably selected so that the mixture obtained will get a consistency and adherence suitable for administration on the body surface, whereby the particle layer remains on the surface. The mixture may suitably have the form of an ointment.

The mixture optionally with additives, may be enclosed between thin permeable support layers of, for example, paper, cotton fabric or an inert plastics material. The fabric must, of course, have such a mesh size that particles cannot pass through the meshes. Instead of fabrics thin films of tissue-acceptable plastics may be used, said films being permeable, optionally through small openings, to liquids and gases.

It is desirable that the mixture be sterile.

The cromoglycic acid, or the pharmaceutically acceptable salt thereof, may be in the form of discrete particles which are physically dispersed through the particles of the macromolecular material or may be incorporated within the particles of the macromolecular material, e.g. may be dispersed through the macromolecular material during its manufacture. When discrete particles of cromoglycic acid, or a pharmaceutically acceptable salt thereof, are used we prefer these particles to have an average particle size of the same order as that of the macromolecular material, e.g. of from about 200 to 300 microns.

Alternatively there may be used fine cromoglycic acid, or a salt thereof, e.g. sodium cromoglycate, of for example mass mean diameter of less than 20 microns, preferably less than 10 microns, e.g. of about 5 microns. This fine material forms a coating on the coarse macromolecular material thereby minimising segregation.

Mixtures accoding to the invention may also be made by absorbing a solution of the cromoglycic acid, or the salt thereof, e.g. an aqueous solution of sodium cromoglycate, on the macromolecular material, followed if necessary by a drying step.

We prefer the ratio of cromoglycic acid or the pharmaceutically acceptable salt thereof, e.g. sodium cromoglycate, to the macromolecular material to be in the range 0.05 to 0.2:1, more preferably 0.05 to 0.1:1, by weight.

According to our invention we also provide a method for the treatment of a condition of the skin of a mammal, e.g. man, cats, dogs and horses, which comprises topical administration of a composition according to the invention to an individual mammal suffering from such a condition.

According to the invention we also provide a method for the treatment of a condition of the skin of a mammal, e.g. man, cats, dogs and horses, which comprises sequential or simultaneous administration of the macromolecular material and cromoglycic acid, or a pharmaceutically acceptable salt thereof, to an individual mammal suffering from such a condition.

Specific conditions in man and other animals which may be treated by the method of the invention include contact dermatitis to a specific allergen, e.g. nickel, chromates, synthetic resins, applied medicaments and other chemicals (Rook A., Wilkinson DS and Ebling FJS 1972 Textbook of Dermatology 2nd Edition Blackwell, Oxford Chapters 14 and 15). Dermatoses which may be treated include contact sensitivity, e.g. to chromium, nickel or an antibiotic, eczemas, drug eruptions, psoriasis, dermatitis herpetiformis, atopic dermatitis, apthous

ulcers, Behçet's syndrome, pemphigus, urticaria, urticaria pigmentosa, the ulcers of Crohn's disease, pyoderma gangrenosum and chronic skin ulcers, notably those affecting older men and women. Specific ulcers which may be mentioned include venous, stasis, decubitis and ischaemic ulcers and bed sores.

The amount of the mixture to be administered will of course vary with the condition to be treated, the animal or patient to be treated and the particular mode of, and formulation used for, administration. When administering the mixture topically, e.g. as granules, the dosage is difficult to control, but will depend in general on the size and condition of the area to be treated.

The wound or lesion to be treated is desirably cleaned, the mixture is then applied, e.g. to a depth of about 0.5 cms, and the area covered and the mixture held in place by a dry dressing. The procedure may be repeated as frequently as is required, e.g. once or twice a day, or more frequently if the wound is draining profusely.

When sequential or simultaneous administration of the macromolecular material and the cromoglycic acid, or the pharmaceutically acceptable salt thereof, is used the ratios and dosages are as described above with respect to the mixtures. We prefer to use the macromolecular material (to clean the wound or lesion) before the cromoglycic acid, or the pharmaceutically acceptable salt thereof, is applied.

The invention therefore also provides a pharmaceutical package comprising at least one dose of macromolecular material and at least one dose of cromoglycic acid, or a pharmaceutically acceptable salt thereof, e.g. sodium cromoglycate. The doses are preferably arranged in the package in a particular order together with written or printed indications or directions, the indications or directions and the manner of packing being such as to provide guidance in relation to and to facilitate the administration of a dose of the macromolecular material and a dose of the cromoglycic acid or the pharmaceutically acceptable salt thereof, in a particular order or combination, e.g. a dose of the former at the same time as a dose of the latter. The package is preferably a sealed package and may comprise a tube, box or chart in or on which the doses are packed. The package is preferably impervious to moisture.

The invention is illustrated by the following Examples.

Example 1

Substantially dry water-insoluble particles of dextran crosslinked with epichlorohydrin (the reaction having been effected in alkaline aqueous solution cf.

British Patent No 974,054) were prepared. The swellability of the particles was such that 1g of dry substance absorbed 2.5g of water. The average particle size was approximately 200 microns. The particles were mixed in a weight proportion of 1.0 to 0.07 with sodium cromoglycate of mass median diameter 200 to 300 microns.

Example 2

Water-insoluble spherical sterile particles of diethylaminoethyl dextran, crosslinked with epichlorohydrin (cf. British Patent No 1,013,585) were prepared. The particle size was approximately 80 microns. The total swellability per g of the dry polymer was more than 2 and less than 10g of water. The swelling rate of the dry particles in physiological sodium chloride solution was 0.4g per g per 5 sec. The particles were mixed in a weight ratio of 1.0 to 0.05 with sodium cromoglycate.

Example 3

Dry spherical particles consisting of crosslinked starch were obtained by a dispersion polymerisation technique in accordance with the method disclosed in British Patent No 974,054; in this method, ethylene dichloride is used as a coherent phase in the dispersion and epichlorohydrin is used as a crosslinking agent. From the spherical particles thus produced, a particle fraction having an average diameter of approximately 100 microns was removed in the dry state by sieving. The water absorption capacity was approximately 4.5g of water per one gram of dry crosslinked starch. The particles were mixed with sodium cromoglycate of the same size in a weight ratio of 1.0 to 0.1 and were sterilised by gamma-radiation. The particles can be completely dissolved by prolonged contact with alpha-amylase in neutral aqueous solution.

Example 4

Particulate material consisting of dextran crosslinked with epichlorohydrin (average particle size approximately 200 microns, swellability 2.5g of water per 1g of dry substance), was mixed with sodium cromoglycate and finely ground bleached cellulose (both of the same size as the cross-linked dextran) in the ratio of crosslinked dextran/cellulose wadding/sodium cromoglycate = 3:1:0.05.

Example 5

A dressing was produced in the following manner: 4 parts by weight of dry spherical particles (consisting of dextran crosslinked with epichlorohydrin in alkaline aqueous solution and having a swellability of 2.5g of water per 1g of dry substance) and 0.2 parts by weight of sodium cromoglycate were added under agitation to 2 parts by weight of bleached fine paper pulp suspended in water. The mixture was stirred strongly with a magnetic agitator for 30 minutes, whereafter the mixture was dried. A flexible, soft, light elastic material was obtained from which pieces could be readily punched and clipped in the desired size. The dressing was also found to possess good tensile strength properties, which could be further enhanced by mixing an inert binder with it. Dressings with other mixing ratios were also prepared.

Example 6

An ointment was made up from the following ingredients:

Polyethyleneglycol having an average molecular weight of about 300 (Macrogol 300)                    14.3%

Polyethyleneglycol having an average molecular weight of about 1500 (Macrogol 1540)                   28.6%

Dry particles of crosslinked dextran having an average particle size of about 200 microns and a swellability in water of 2.5g of water per gram of dry substance (prepared according to British Patent No 974,054 by crosslinking dextran with epichlorohydrin in alkaline solution)                                            52.1%

Sodium cromoglycate                                  5.00%

                                                   100.0%

                                              by weight

The two polyethylene glycol products were first melted together to form a melt whereupon the dry particles of crosslinked dextran and the sodium cromoglycate were added to the melt with stirring. The mixture was then cooled to about 20°C.

What we claim is:-

1. A mixture of cromoglycic acid, or a pharmaceutically acceptable salt thereof, and substantially dry particles of a water-insoluble hydrophilic macromolecular material capable of undergoing limited swelling in fluid to form discrete gel particles, the swellability of the macromolecular material being such that the particles can absorb low molecular weight constitutents of blood plasma but cannot absorb to any material extent fibrinogen or other substances of the same or higher molecular weight.

2. A mixture according to Claim 1, wherein the macromolecular material has a high water swelling rate and a water absorption ability of from 1.5 to 10g of water per gram of dry macromolecular material.

3. A mixture according to Claim 1 or 2, wherein the macromolecular material is dextran cross linked with epichlorhydrin.

4. A mixture according to any one of the preceding claims, wherein the dry particles of macromolecular material have an average particle size in the range 50 to 300 microns.

5. A mixture according to any one of the preceding claims comprising sodium cromoglycate.

6. A mixture according to any one of the preceding claims, wherein the cromoglycic acid, or the pharmaceutically acceptable salt thereof, is in particulate form and these particles have an average particle size of the same order as that of the macromolecular material.

7.    A mixture according to any one of Claims 1 to 5, wherein the cromoglycic acid, or the pharmaceutically acceptable salt thereof, has a mass median diameter of less than 10 microns and the macromolecular material has an average particle size of 200 to 300 microns.

8.    A mixture according to any one of the preceding claims, wherein the ratio of the cromoglycic acid, or the pharmaceutically acceptable salt thereof, to the macromolecular material is from 0.05 to 0.2:1 by weight.

9.    A pack comprising a mixture according to any one of the preceding claims, or its components, and one or more protective layers for retaining the mixture when it is applied to a wound.

10.    A method of making a mixture according to any one of Claims 1 to 8, which comprises mixing dry particles of the ingredients or absorbing a solution of cromoglycic acid, or a pharmaceutically acceptable salt thereof, onto the macromolecular material.